(19) Europäisches Patentamt
European Patent Office
Office européen des brevets

(11) **EP 4 418 987 B1**

(12) **EUROPEAN PATENT SPECIFICATION**

(45) Date of publication and mention
of the grant of the patent:
**18.06.2025  Bulletin 2025/25**

(21) Application number: **22808667.4**

(22) Date of filing: **19.10.2022**

(51) International Patent Classification (IPC):
*A61B 5/00* (2006.01)      *A61B 5/347* (2021.01)
*A61B 5/08* (2006.01)

(52) Cooperative Patent Classification (CPC):
**A61B 5/7246; A61B 5/0816; A61B 5/347;**
**A61B 5/686**

(86) International application number:
**PCT/EP2022/079062**

(87) International publication number:
**WO 2023/066975 (27.04.2023 Gazette 2023/17)**

(54) **METHOD AND MEDICAL DEVICE FOR DETERMINING A PERIODICITY OF A PHYSIOLOGICAL SIGNAL**

VERFAHREN UND MEDIZINISCHE VORRICHTUNG ZUR BESTIMMUNG EINER PERIODIZITÄT EINES PHYSIOLOGISCHEN SIGNALS

PROCÉDÉ ET DISPOSITIF MÉDICAL POUR DÉTERMINER UNE PÉRIODICITÉ D'UN SIGNAL PHYSIOLOGIQUE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **22.10.2021  US 202163270781 P**
**12.11.2021  EP 21207871**

(43) Date of publication of application:
**28.08.2024  Bulletin 2024/35**

(73) Proprietor: **BIOTRONIK SE & Co. KG**
**12359 Berlin (DE)**

(72) Inventors:
• **GUHANIYOGI, Shayan**
**Portland, Oregon 97206 (US)**
• **DE VOIR, Christopher S.**
**Portland, Oregon 97201 (US)**

(74) Representative: **Biotronik Corporate Services SE**
**Corporate Intellectual Property**
**Sieversufer 7-9**
**12359 Berlin (DE)**

(56) References cited:
**GB-A- 2 020 043          US-A- 5 243 993**
**US-A- 5 365 934          US-A1- 2003 185 449**
**US-A1- 2014 073 864      US-A1- 2014 073 951**
**US-A1- 2014 073 960      US-A1- 2014 073 974**
**US-B2- 7 076 109**

• **AYANCE ADAN TORRALBA ET AL: "Low-cost microcontrolled based wireless heart rate and oxygen saturation monitor", 2018 INTERNATIONAL CONFERENCE ON ELECTRONICS, COMMUNICATIONS AND COMPUTERS (CONIELECOMP), IEEE, 21 February 2018 (2018-02-21), pages 176 - 180, XP033341101, DOI: 10.1109/ CONIELECOMP.2018.8327195**

**Description**

Embodiments of the present disclosure relate to a method for determining a periodicity of a physiological signal, a machine-readable medium for executing the method, and a medical device for determining a periodicity of a physiological signal. Embodiments of the present disclosure relate more particularly to an efficient autocorrelation for detecting periodicity of signals in a low-power active implantable medical device.

Active implantable medical devices (AIMDs) can measure various physiological signals for which the periodicity of such signals has clinical importance. For example, the periodicity of an ECG signal is heart rate; the periodicity of a respiratory signal is respiratory rate; the periodicity of an activity signal such as walking is the walking cadence. Reporting these periodicities provides clinicians with important information about the status of a patient's health. Furthermore, reporting the lack of periodicity in such signals also provides important information (e.g., atrial fibrillation, sleep apnea, imbalanced gait etc.). AIMDs therefore employ methods to measure the periodicity of physiological signals and report them to clinicians or end-users.

Due to the low-power nature of AIMDs, the methods available for measuring the periodicity of signals are limited. Most frequently, the periodicity of a signal is measured by using thresholding techniques to detect peaks of the signal, and then measuring the elapsed time between the peaks. However, this method is subject to various errors (e.g., contamination by noise, improper threshold selection, underdetection/overdetection of peaks, etc.) which require delicate fine-tuning in order to achieve acceptable performance.

On the other hand, more robust methods for measuring the periodicity of a signal, such as spectral methods, are too computationally intensive for a low-power AIMD.

The published paper "Low-cost microcontrolled based wireless heart rate and oxygen saturation monitor" from Adán Torralba Ayance et al. (Electronic ISSN: 2474-9044) describes the design and development of a wireless heart rate and oxygen saturation monitor based on low cost microcontrollers, wherein pulse oximetry is used to measure these vital signs.

US 2003/0185449 A1 describes the fast automatic finding of periodicities in sequential digital binary data, and more particularly, a system and method of automatically and expeditiously determining the optimal or dominant periodicity in bitonal images, for use in data compression and/or other applications.

In light of the above, a method for determining a periodicity of a physiological signal, a machine-readable medium for executing the method, and a medical device for determining a periodicity of a physiological signal that overcome at least some of the problems in the art are beneficial.

It is an object of the present invention to provide a computer-implemented method for determining a periodicity of a physiological signal according to claim 1, a machine-readable medium for executing the method according to claim 11, and a medical device according to claim 12 for determining a periodicity of a physiological signal that can detect the periodicity efficiently and/or with less processing resources. It is another object of the present disclosure to provide an efficient autocorrelation method that can be implemented in a low-power AIMD.

The objects are solved by the features of the independent claims. Preferred embodiments are defined in the dependent claims.

The definition of the plurality of target periodicities, which are periodicities of interest, allows the autocorrelation to be calculated only at specific lag points. This an improvement of computational efficiency. Furthermore, the conversion of the sample to a binary value allows a reduction in memory usage, improves the efficiency of the autocorrelation calculation, and allows real-time calculation of the autocorrelation.

According to some embodiments, which can be combined with other embodiments described herein, the physiological signal can be a respiratory signal or an electrocardiogram (ECG) signal. However, the present disclosure is not limited thereto and other physiological signals which for which the periodicity of such signals has clinical importance.

According to some embodiments, which can be combined with other embodiments described herein, the plurality of target periodicities is in units of cycles per minutes or cycles per second (Hz), however providing the target periodicities in units of cycles per second is preferred, as it can simplify the processing at later stages of the algorithm. Furthermore, the discrete range of probable periodicities that are of interest for the given physiological signal can increase an efficiency in the autocorrelation.

According to some embodiments, which can be combined with other embodiments described herein, a lower cutoff frequency of the bandpass filter is below a minimum periodicity of the plurality of target periodicities and an upper cutoff frequency of the bandpass filter is above a maximum periodicity of the plurality of target periodicities.

Accordingly, the filter will consequently suppress any DC/baseline offset in the physiological signal, and any frequencies above the maximum periodicity of interest.

The Boolean data type is a data type that has one of two possible values, namely True and False. This conversion to a binary representation provides two main advantages. First, it makes the autocorrelation calculation more efficient. Second, it drastically reduces the memory required by the algorithm. For example, if each signal sample was originally represented by one byte and can now be now represented by a single bit, that is an 8-fold reduction of the required memory.

According to some embodiments, which can be combined with other embodiments described herein, the method further includes storing the binary values in a circular buffer. The circular buffer is a data structure that uses a single, fixed-size buffer as if it were connected end-to-end.

Preferably, each element of the circular buffer is a bit. In other words, the circular buffer can be a circular bit buffer.

According to some embodiments, which can be combined with other embodiments described herein, the autocorrelation is calculated using an exclusive NOR (XNOR) function which produces a value of 1 when both binary inputs are equal. Using the XNOR function on two bits to calculate the autocorrelation has less computational expense than, for example, multiplying two integer or floating-point samples together to calculate autocorrelation. The use of binary values consequently not only saves memory, but also improves the efficiency of the autocorrelation calculation.

According to some embodiments, which can be combined with other embodiments described herein, the autocorrelation is calculated for lags that correspond to the plurality of target periodicities.

Autocorrelation is calculated by correlating a signal with many delayed, or "lagged", versions of itself. Generally, for a real discrete time signal $y[k]$, the autocorrelation $\alpha$ at a particular lag $l$ can be calculated as:

$$\alpha(l) = \sum_k y[k] * y[k - l]$$

In other words, to calculate the autocorrelation $\alpha$ at a particular lag $l$, each sample in the signal is multiplied by the $l^{th}$ prior sample, and the results of each multiplication are summed. The period of the signal can then be calculated by finding the first lag $l$ at which the maximum $\alpha$ occurs.

According to some embodiments, which can be combined with other embodiments described herein, the autocorrelation is a real-time autocorrelation. For example, at the beginning of the algorithm, all $\alpha(l)$ are initialized to zero. Thereafter, each time a new sample is added to the circular buffer, $\alpha(l)$ is updated using the equation above. The result is a real-time autocorrelation of the input signal.

According to some embodiments, which can be combined with other embodiments described herein, the method further includes stopping the calculating of the autocorrelation after a predetermined time ("observation period") has elapsed. This observation period can be as long as needed, limited only by device constraints or requirements of the clinical application.

According to some embodiments, which can be combined with other embodiments described herein, determining a periodicity of the physiological signal based on the calculated autocorrelation includes:

- finding a first peak of the calculated autocorrelation in a direction from a shortest lag to a longest lag; and
- defining the first peak as the periodicity of the physiological signal.

Using the first peak of autocorrelation to define the periodicity is beneficial due to the repetitive nature of the autocorrelation output. Briefly, if a signal is periodic at some lag $l$, then the autocorrelation of that signal will produce a local peak at $l$, but will also produce local peaks at $2l$, $3l$, $4l$, etc. (i.e., all integer multiples of $l$). Consequently, the first $l$ at which there is a peak corresponds to the true periodicity of the signal.

According to some embodiments, which can be combined with other embodiments described herein, the method further includes:

- comparing the first peak to a threshold; and
- defining the first peak as the periodicity of the physiological signal only if the first peak or a difference between the first peak and a minimum element of the calculated autocorrelation exceeds the threshold.

Comparing the first peak to the threshold ensures that it actually represents a strong periodic component of the signal rather than, for example, random noise.

According to some embodiments, which can be combined with other embodiments described herein, the method further includes transmitting the determined periodicity of the physiological signal to a mobile device using wireless communication.

Preferably, the mobile terminal of the patient and/or the mobile terminal of the physician is selected from the group including (or consisting of) a smartphone, a personal digital assistant, a tablet, a notebook, a smart watch, any device with a web browser, and smart glasses.

Preferably, the wireless communication takes place via at least one communications network, such as a local area network (LAN), a Bluetooth connection, an NFC connection and/or a wide area network (WAN), in particular the Internet.

Embodiments are also directed at systems/devices for carrying out the disclosed methods and include system/-

device aspects for performing each described method aspect. These method aspects may be performed by way of hardware components, a computer programmed by appropriate software, by any combination of the two or in any other manner. Furthermore, embodiments according to the invention are also directed at methods for operating the described device/system. It includes method aspects for carrying out every function of the device/system.

According to another independent aspect of the present disclosure, a machine-readable medium is provided. The machine-readable medium includes instructions executable by one or more processors to implement the method for determining a periodicity of a physiological signal of any of claims **1-10.**

The (e.g. non-transitory) machine readable medium may include, for example, optical media such as CD-ROMs and digital video disks (DVDs), and semiconductor memory devices such as Electrically Programmable Read-Only Memory (EPROM), and Electrically Erasable Programmable Read-Only Memory (EEPROM). The machine-readable medium may be used to tangibly retain computer program instructions or code organized into one or more modules and written in any desired computer programming language. When executed by, for example, one or more processors such computer program code may implement one or more of the methods described herein.

According to another independent aspect of the present disclosure, a medical device according to claim **12** for determining a periodicity of a physiological signal is provided. The medical device includes the machine-readable medium of the embodiments of the present disclosure. According to some embodiments, which can be combined with other embodiments described herein, the medical device is an active implantable medical device.

So that the manner in which the above recited features of the present disclosure can be understood in detail, a more particular description of the disclosure, briefly summarized above, may be had by reference to embodiments. The accompanying drawings relate to embodiments of the disclosure and are described in the following:

Fig. 1    shows a flowchart of a method for determining a periodicity of a physiological signal according to embodiments of the present disclosure;

Fig. 2    shows a simulated respiratory signal with a DC offset and high-frequency noise (top); the signal after preprocessing with a bandpass filter (middle); the signal after converting to a binary representation (bottom); and

Fig. 3    shows a circular bit buffer storing the binary representation of the physiological signal according to embodiments of the present disclosure.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations, whereas the invention is defined by the appended claims.

Many physiological signals are periodic in nature, and the periodicity of these signals is often of clinical importance. Active implantable medical devices (AIMDs) may measure the periodicity of such signals. The present disclosure describes a new approach that uses efficient autocorrelation and can therefore be implemented within the memory and computational constraints of AIMDs, and still provide the benefits of an accurate autocorrelation-based method to calculate the periodicity of a signal.

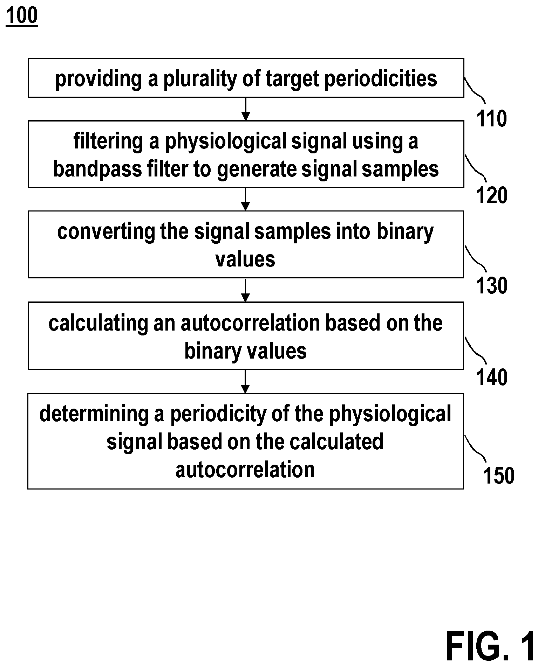

Fig. 1 shows a flowchart of a method 100 for determining a periodicity of a physiological signal according to embodiments of the present disclosure.

The method includes in block 110 providing a plurality of target periodicities; in block 120 filtering a physiological signal using a bandpass filter to generate signal samples; in block 130 converting the signal samples into binary values; in block 140 calculating an autocorrelation based on the binary values; and in block 150 determining a periodicity of the physiological signal based on the calculated autocorrelation.

A detailed example of the method for determining a periodicity of a physiological signal is explained below.

The embodiment described here will consider detecting the periodicity of a respiratory signal (i.e., detecting the respiratory rate). However, it will be apparent to those skilled in the art that the techniques described here can be applied with appropriate modifications to other signal types (e.g., ECG, activity, etc.).

Determining the periodicities of interest

To gain efficiency in the proposed autocorrelation method, the algorithm is developed around a known, discrete range of probable periodicities that are of interest for the given physiological signal. Using a respiratory signal as an example, it is known that typical respiratory rates can range between 10 to 25 breaths per minute. Furthermore, from a clinical perspective, it may only be necessary to discriminate between respiratory rates at a resolution of 1 breath per minute. Consequently, the periodicities of interest for a respiratory rate autocorrelation algorithm could be [10, 11, 12, ...,

25] breaths per minute.

In more general terms, let **P** denote all periodicities of interest:

$$P = \begin{pmatrix} p_1 \\ p_2 \\ \vdots \\ p_n \end{pmatrix}$$

where $n$ is the total number of periodicities, and $p_i$ denotes the $i^{th}$ periodicity in **P**. To simplify the math at later stages of the algorithm, **P** should be in units of cycles per second (Hz). Therefore, in the example above, [10, 11, 12, ..., 25] breaths per minute would be converted to $[\frac{10}{60}, \frac{11}{60}, \frac{12}{60}, \ldots, \frac{25}{60}]$ breaths per second when used as **P**.

As will be made clear later, having less periodicities of interest (smaller $n$) will make the algorithm more efficient. However, this is a tradeoff that should be balanced against clinical requirements. For example, if the proposed algorithm were to be applied to an ECG signal to detect heart rate, clinical requirements may dictate that there should be more periodicities of interest, for example 20 to 240 beats per minute, in 5 beat per minute increments.

Signal preprocessing

Once **P** is known for a given application, it should be used to define a bandpass filter that can be used to pre-process the signal of interest in real-time. Specifically, the lower cutoff frequency of this bandpass filter should be set somewhere below the minimum value in **P**, and the upper cutoff frequency should be set somewhere above the maximum value in **P**. The filter will consequently suppress any DC/baseline offset in the signal, and any frequencies above the maximum periodicity of interest. Due to the real-time requirement, this filter is best suited to be implemented as a hardware filter on the AIMD, however it may also be done in software.

Converting the signal samples to binary values

As the incoming signal is filtered as described above, each filtered signal sample is converted to a Boolean by comparing it to a value of zero. If the filtered sample is positive (greater than zero), it is given a value of True, and otherwise it is given a value of False. Consequently, the sample that may have originally been represented in the device's software as an integer or floating point data type can now be represented by a single bit: 1 for True and 0 for False.

This conversion to a binary representation provides two main advantages. First, it makes the autocorrelation calculation more efficient, as will be described in more detail later. Second, it drastically reduces the memory required by the algorithm. For example, if each signal sample was originally represented by one byte and can now be now represented by a single bit, that is an 8-fold reduction of the required memory.

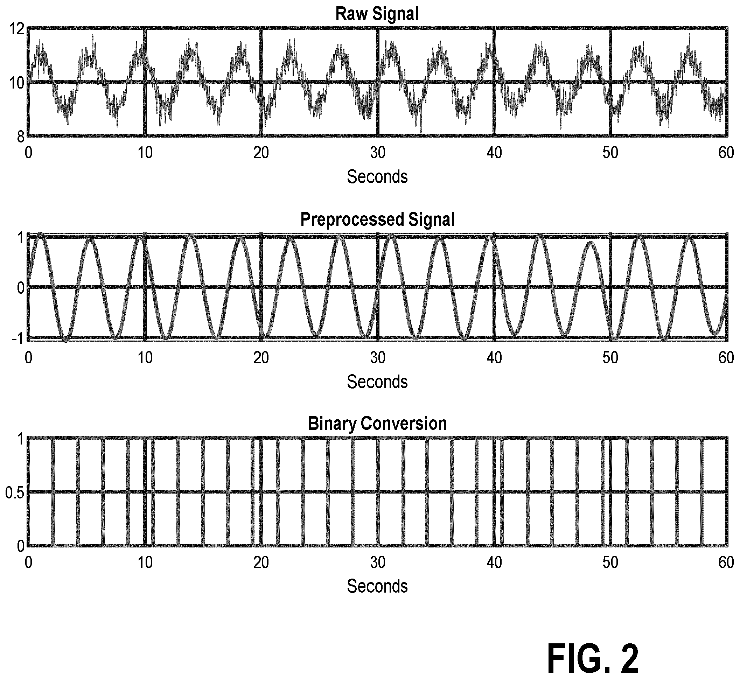

Fig. 2 visually depicts the signal preprocessing and binary conversion steps for a simulated respiratory signal. The top graph of Fig. 1 shows a simulated respiratory signal with a DC offset and high-frequency noise. The middle graph shows the signal after preprocessing with a bandpass filter to remove the DC offset and high-frequency noise. The bottom graph shows the signal after converting to a binary representation by comparing the preprocessed signal (middle) to a value of zero. Note that the binary representation maintains the periodicity of the original signal.

Storing the binary values in a circular bit buffer

After generating a binary value for the filtered signal sample, this binary value is subsequently put into a circular bit buffer. Let $p_i = P[i]$ denote the $i^{th}$ periodicity in **P**. The length of this circular bit buffer, $m$, must be equal to:

$$m = ceil\left(\frac{f_s}{\min_i(\boldsymbol{P}[i])}\right) + 1 \qquad Eqn.\,1$$

where $f_s$ is the sampling rate of the signal, and $\min_i(\boldsymbol{P}[i])$ is the minimum periodicity of interest in **P**.

Using the example above, the minimum periodicity of interest was $\frac{10}{60}$ breaths per second. If the signal was acquired at a sampling rate of $f_s$ = 32Hz, then the length $m$ of the circular buffer in this example would be:

$$ceil\left(\frac{32}{\frac{10}{60}}\right) + 1 = 193 \; bits$$

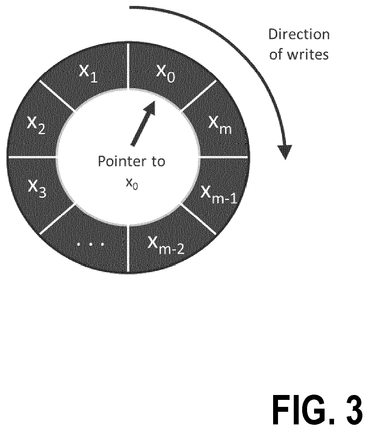

The most recent binary value placed into the circular bit buffer is denoted as $x_0$, the previous binary value is denoted as $x_1$, the value prior to that as $x_2$, and so on until $x_m$ (the oldest value contained in the buffer). The circular bit buffer can then be visualized as shown in Fig. 3. In particular, Fig. 3 depicts the circular bit buffer storing the binary representation of the signal from the most recent sample $x_0$ to the oldest sample $x_m$, where $m$ is the length of the buffer as calculated using the equation above. A pointer is kept keeping track of the location of $x_0$ within the buffer.

Note that in most applications of circular buffers each element in the buffer is a byte, and the buffer operations are byte-wise operations. In this method however, each element in the buffer is a bit, and the buffer operations therefore uses bit-wise operations.

Calculating autocorrelation

Autocorrelation is calculated by correlating a signal with many delayed, or "lagged", versions of itself. Generally, for a real discrete time signal $y[k]$, the autocorrelation $\alpha$ at a particular lag $l$ can be calculated as:

$$\alpha(l) = \sum_k y[k] * y[k - l] \qquad Eqn. \, 2$$

In other words, to calculate the autocorrelation $\alpha$ at a particular lag $l$, each sample in the signal is multiplied by the $l^{th}$ prior sample, and the results of each multiplication are summed. The period of the signal can then be calculated by finding the first lag $l$ at which the maximum $\alpha$ occurs. This is because when a periodic signal is lagged by an amount equal to its period (or any integer multiple of its period) there is still a high overlap between the lagged signal and the original signal. Conversely, when a periodic signal is lagged by an amount equal to half of its period, it produces the worst overlap between the lagged signal and the original signal.

It can be appreciated that the circular buffer described earlier contains binary representations of up to $m$ samples prior to the current sample (i.e., contains samples $x_0$ through $x_m$). Consequently, the autocorrelation $\alpha$ at a particular lag $l$ (for $0 \le l \le m$) can be calculated from the circular buffer as:

$$\alpha(l) \mathrel{+}= XNOR(x_0, x_l) \qquad Eqn. \, 3$$

where XNOR is the exclusive NOR function, and "+=" is the addition assignment operator.

XNOR produces a value of 1 only when both inputs are equal, and therefore acts as a correlation operator between $x_0$ and $x_l$. Note that the use of XNOR is possible due to the use of binary values in the circular buffer. Using the XNOR function on two bits to calculate the autocorrelation has less computational expense than, for example, multiplying two integer or floating-point samples together to calculate autocorrelation. The use of binary values in the circular buffer consequently not only saves memory, but also improves the efficiency of the autocorrelation calculation in this method.

The addition assignment operator is used to indicate that the right side of the equation is added to the existing value of $\alpha(l)$ each time the calculation is made. At the beginning of the algorithm, all $\alpha(l)$ are initialized to zero. Thereafter, each time a new sample is added to the circular buffer, $\alpha(l)$ is updated using the equation above. The result is a real-time autocorrelation of the input signal.

While $\alpha(l)$ could be calculated for all $l$ from 0 to $m$, the periodicities of interest $P$ have been defined Consequently, $\alpha(l)$ only needs to be calculated for the lags $l$ that correspond to the periodicities of interest $P$, further reducing the computational complexity. These specific lags of interest, $L$, can be calculated using the equation:

$$L = \begin{pmatrix} l_1 \\ l_2 \\ \vdots \\ l_n \end{pmatrix} = round\left(\frac{f_s}{P}\right) = round\begin{pmatrix} f_s/p_1 \\ f_s/p_2 \\ \vdots \\ f_s/p_n \end{pmatrix} \qquad Eqn. \, 4$$

The round function is applied because $\frac{f_s}{P}$ may not always produce an integer lag. Then, using the lags of interest

$L$, one can define the autocorrelations of interest, $R$, as:

$$R = \begin{pmatrix} r_1 \\ r_2 \\ \vdots \\ r_n \end{pmatrix} = \alpha(L) = \begin{pmatrix} \alpha(l_1) \\ \alpha(l_2) \\ \vdots \\ \alpha(l_n) \end{pmatrix} \qquad Eqn.\,5$$

Continuing the previous example, where $P = [\frac{10}{60}, \frac{11}{60}, \frac{12}{60}, \ldots, \frac{25}{60}]$ breaths per second and $f_s = 32$Hz, using the equations above results in the lags of interest $L = [192, 175, 160, ..., 77]$, and consequently the autocorrelations of interest $R = [\alpha(192), \alpha(175), \alpha(160), ... , \alpha(77)]$.

Observation period

As described earlier, when the algorithm is initiated, all $\alpha(l)$, and thus $R$, are initialized with zero. Calculations for $R$ should then only begin once the circular buffer has filled for the first time (so as not to calculate $R$ using uninitialized values in the circular buffer).

Calculations for $R$ should end after a certain observation period. This observation period can be as long as needed, limited only by device constraints or requirements of the clinical application.

Finding the periodicity

After the autocorrelations of interest $R$ have been calculated for the desired observation period, the periodicity of the signal can be determined by finding the first peak of $R$ in the direction of the shortest lag to the longest lag.

Note that the concept of using the first peak of autocorrelation to define the periodicity is beneficial due to the repetitive nature of the autocorrelation output. Briefly, if a signal is periodic at some lag $l$, then the autocorrelation of that signal will produce a local peak at $l$, but will also produce local peaks at $2l$, $3l$, $4l$, etc. (i.e. all integer multiples of $l$). Consequently, the first $l$ at which there is a peak corresponds to the true periodicity of the signal.

Note that in the example above, $R$ was ordered from longest lag to shortest lag. However, in practice the order of $R$ in memory does not matter, so long as the algorithm to find the first peak from shortest lag to longest lag knows this order. For example, if $R$ is stored in order of longest lag to shortest lag as above, the algorithm can work "backwards" from the end of the $R$ array to find the first peak.

Once the location of the first peak in $R$ is found, it can be used to calculate the periodicity. Note that $R$, $L$, and $P$ are all vectors of equal length $n$. Let $r_i = R[i]$ denote the $i^{th}$ autocorrelation value in $R$. Let $\underset{i}{\text{firstpeak}}()$ be the function that outputs the first peak value of an array, and $\underset{i}{\text{arg firstpeak}}()$ be the function that outputs the index of the first peak of an array.

Then, the periodicity of the signal is found as:

$$Periodicity = P\left[\underset{i}{\text{arg firstpeak}}(R[i])\right] \qquad Eqn.\,6$$

In the example above, there was $R = [\alpha(192), \alpha(175), \alpha(160), ... , \alpha(77)]$ and $P = [\frac{10}{60}, \frac{11}{60}, \frac{12}{60}, \ldots, \frac{25}{60}]$ breaths per second. Now if $\alpha(175)$ was found to be the first peak in $R$, that would correspond to the 2$^{nd}$ element of $R$ (in other words $\underset{i}{\text{arg firstpeak}}(R[i]) = 2$). Consequently, the periodicity would be determined as $P[2] = \frac{11}{60}$ breaths per second.

However, a special consideration can be made when using the first peak of $R$. As an example, for a truly non-periodic signal (e.g., random noise), all $\alpha(l)$ would be small (close to zero). Therefore, simply picking the first peak from an array of small $\alpha(l)$ could be an incorrect determination of periodicity in a noisy signal when there is none. Consequently, the first peak of $R$ should be compared to a threshold $\tau$ to ensure that it actually represents a strong periodic component of the signal. Note that the elements of $R$ can take on values between 0 and the total number of samples collected during the observation period, *N*. The value of $\tau$ can therefore be any value between 0 and *N*. As $\tau$ is set closer to *N,* more stringent criteria are set for the strength of the periodicity.

In other words, in order to report a periodicity for the signal, the following condition must be met:

$$\operatorname*{firstpeak}_{i}(\boldsymbol{R}[i]) > \tau \qquad Eqn.\,7$$

Alternatively, the threshold can be based on the difference between the first peak of *R* and the minimum element of *R.* This is because in highly periodic signals the difference between the maximum and minimum $\alpha(l)$ would be large, while for non-periodic signals such as random noise, that difference would be very small. In other words, to report a periodicity, the following condition must be met:

$$\left(\operatorname*{firstpeak}_{i}(\boldsymbol{R}[i]) - \min_{i}(\boldsymbol{R}[i])\right) > \tau \qquad Eqn.\,8$$

where again the value of $\tau$ can be between 0 and *N.* In either case, if the condition is not met, the algorithm may report the lack of periodicity in the signal (if the lack of periodicity is also of clinical importance).

The above method can be summarized as below:

1) Initialization:

   a. Define the periodicities of interest *P* of a given physiological signal based on the clinical application
   b. Implement a bandpass filter based on the lowest and highest periodicities in *P*
   c. Allocate a circular bit buffer of length *m* using Eqn. 1
   d. Calculate the lags of interest *L* from *P* using Eqn. 4
   e. Initialize a zero-vector *R* of length equal to *P*

2) Real-time autocorrelation. For each incoming signal sample:

   a. Pass the sample through the bandpass filter defined above
   b. Compare the filtered sample to a value of zero to convert the sample to a binary value

      i. If the filtered sample >= 0, represent the sample as a binary 1
      ii. If the filtered sample < 0, represent the sample as a binary 0

   c. Store the binary representation of the sample in the circular bit buffer
   d. Update *R* using Eqn. 5 and Eqn. 3

3) After the observation period has ended, calculate the periodicity of the signal:

   a. Find the first peak of *R* in the direction of the shortest lag to the longest lag
   b. Compare the value of the peak to a threshold $\tau$ using either Eqn. 7 or Eqn. 8
   c. If the condition of Eqn. 7 or Eqn. 8 is met (depending on which one is used), report a periodicity for the signal using Eqn. 6
   d. If the conditions are not met, the algorithm may choose to report the lack of periodicity, if this is of clinical importance.

The definition of periodicities of interest *P* allows the autocorrelation *R* to be calculated only at specific lag points *L.* This an improvement of computational efficiency. Furthermore, the conversion of the sample to a binary value and storage into a circular bit buffer allows a reduction in memory usage, improves the efficiency of the autocorrelation calculation by the use of the XNOR function, and allows real-time calculation of the autocorrelation.

## Claims

1.  A computer-implemented method for determining a periodicity of a physiological signal, comprising:

Providing (110) a plurality of target periodicities from a discrete range of probable periodicities of interest for a physiological signal;

Filtering (120) the plurality of target periodicities of the physiological signal using a bandpass filter to generate signal samples, wherein a lower cutoff frequency of the bandpass filter is below a minimum periodicity of the plurality of target periodicities and an upper cutoff frequency of the bandpass filter is above a maximum periodicity of the plurality of target periodicities;

Converting (130) the signal samples into binary values, comprising: converting each signal sample to a Boolean by comparing the signal sample to zero, wherein the signal sample is given a value of True if the signal sample is greater than zero and a value of False otherwise; and representing the signal sample by a single bit according to its Boolean;

Calculating (140) an autocorrelation (R) based on the binary values; and

Determining (150) a periodicity (P) of the physiological signal based on the calculated autocorrelation;

wherein determining a periodicity of the physiological signal based on the calculated autocorrelation comprises:

Finding a first peak of the calculated autocorrelation in a direction from a shortest lag to a longest lag; and
Defining the first peak as the periodicity of the physiological signal.

2. The method of claim 1, wherein the plurality of target periodicities is in units of cycles per minutes or cycles per second.

3. The method of claim 1, further comprising storing the binary values in a circular buffer.

4. The method of claim 3, wherein each element of the circular buffer is a bit.

5. The method of claim 1, wherein the autocorrelation is calculated using an exclusive NOR function which produces a value of 1 when both binary inputs are equal.

6. The method of claim 1, wherein the autocorrelation is calculated for lags that correspond to the plurality of target periodicities.

7. The method of claim 1, wherein the autocorrelation is a real-time autocorrelation.

8. The method of claim 1, further comprising stopping the calculating of the autocorrelation after a predetermined time has elapsed.

9. The method of claim 1, further comprising:

comparing the first peak to a threshold; and
defining the first peak as the periodicity of the physiological signal only if the first peak or a difference between the first peak and a minimum element of the calculated autocorrelation exceeds the threshold.

10. The method of claim 9, further comprising transmitting the determined periodicity of the physiological signal to a mobile device using wireless communication.

11. A machine-readable medium, comprising instructions executable by one or more processors to implement the method for determining a periodicity of a physiological signal of any one of claims 1 to 10.

12. A medical device for determining a periodicity of a physiological signal, comprising the machine-readable medium of claim 11.

13. The medical device of claim 12, wherein the medical device is an active implantable medical device (AIMD).

**Patentansprüche**

1. Ein computerimplementiertes Verfahren zur Bestimmung der Periodizität eines physiologischen Signals, das Folgendes umfasst:

Bereitstellen (110) einer Vielzahl von Zielperiodizitäten aus einem diskreten Bereich wahrscheinlicher Periodizitäten von Interesse für ein physiologisches Signal;

Filtern (120) der Vielzahl von Zielperiodizitäten des physiologischen Signals unter Verwendung eines Bandpassfilters, um Signalabtastwerte zu erzeugen, wobei eine untere Grenzfrequenz des Bandpassfilters unter einer minimalen Periodizität der Vielzahl von Zielperiodizitäten liegt und eine obere Grenzfrequenz des Bandpassfilters über einer maximalen Periodizität der Vielzahl von Zielperiodizitäten liegt;

Umwandeln (130) der Signalabtastwerte in binäre Werte, umfassend: Umwandeln jedes Signalabtastwerts in einen Booleschen Wert durch Vergleichen des Signalabtastwerts mit Null, wobei dem Signalabtastwert ein Wert Wahr gegeben wird, wenn der Signalabtastwert größer als Null ist, und andernfalls ein Wert Falsch;

und Darstellen des Signalabtastwerts durch ein einzelnes Bit entsprechend seinem Booleschen Wert;

Berechnen (140) einer Autokorrelation (R) auf der Grundlage der binären Werte; und

Bestimmen (150) einer Periodizität (P) des physiologischen Signals auf der Grundlage der berechneten Autokorrelation;

wobei die Bestimmung einer Periodizität des physiologischen Signals auf der Grundlage der berechneten Autokorrelation umfasst:

Auffinden einer ersten Spitze der berechneten Autokorrelation in einer Richtung von einer kürzesten Verzögerung zu einer längsten Verzögerung; und

Definieren der ersten Spitze als die Periodizität des physiologischen Signals.

2. Das Verfahren nach Anspruch 1, wobei die Vielzahl der Zielperiodizitäten in Einheiten von Zyklen pro Minute oder Zyklen pro Sekunde angegeben ist.

3. Das Verfahren nach Anspruch 1, das ferner das Speichern der Binärwerte in einem Ringpuffer umfasst.

4. Das Verfahren nach Anspruch 3, wobei jedes Element des Ringpuffers ein Bit ist.

5. Das Verfahren nach Anspruch 1, wobei die Autokorrelation unter Verwendung einer exklusiven NOR-Funktion berechnet wird, die einen Wert von 1 erzeugt, wenn beide binären Eingänge gleich sind.

6. Das Verfahren nach Anspruch 1, wobei die Autokorrelation für Verzögerungen berechnet wird, die der Vielzahl von Zielperiodizitäten entsprechen.

7. Das Verfahren nach Anspruch 1, wobei die Autokorrelation eine Echtzeit-Autokorrelation ist.

8. Das Verfahren nach Anspruch 1 umfasst ferner das Beenden der Berechnung der Autokorrelation nach Ablauf einer vorbestimmten Zeit.

9. Das Verfahren nach Anspruch 1 umfasst ferner:

Vergleichen der ersten Spitze mit einem Schwellenwert; und

Definieren der ersten Spitze als Periodizität des physiologischen Signals nur dann, wenn die erste Spitze oder eine Differenz zwischen der ersten Spitze und einem minimalen Element der berechneten Autokorrelation den Schwellenwert überschreitet.

10. Das Verfahren nach Anspruch 9 umfasst ferner das Übertragen der bestimmten Periodizität des physiologischen Signals an ein mobiles Gerät unter Verwendung drahtloser Kommunikation.

11. Ein maschinenlesbares Medium mit Befehlen, die von einem oder mehreren Prozessoren ausgeführt werden können, um das Verfahren zur Bestimmung einer Periodizität eines physiologischen Signals nach einem der Ansprüche 1 bis 10 zu implementieren.

12. Ein medizinisches Gerät zur Bestimmung der Periodizität eines physiologischen Signals, umfassend das maschinenlesbare Medium nach Anspruch 11.

13. Das medizinische Gerät nach Anspruch 12, wobei das medizinische Gerät ein aktives implantierbares medizinisches Gerät (AIMD) ist.

**Revendications**

1. Procédé mis en œuvre par ordinateur destiné à déterminer une périodicité d'un signal physiologique, comprenant les étapes consistant à :

   fournir (110) une pluralité de périodicités cibles depuis une plage discrète de périodicités d'intérêt probables pour un signal physiologique ;
   filtrer (120) la pluralité de périodicités cibles du signal physiologique en utilisant un filtre passe-bande pour générer des échantillons de signal, dans lequel une fréquence de coupure basse du filtre passe-bande est inférieure à une périodicité minimale de la pluralité de périodicités cibles et une fréquence de coupure haute du filtre passe-bande est supérieure à une périodicité maximale de la pluralité de périodicités cibles ;
   convertir (130) les échantillons de signaux en valeurs binaires, comprenant les étapes consistant à : convertir chaque échantillon de signal en booléenne en comparant l'échantillon de signal à zéro, dans lequel l'échantillon de signal se voit attribuer une valeur de Vrai si l'échantillon de signal est supérieur à zéro et une valeur de Faux dans le cas contraire ; et représenter l'échantillon de signal par un bit unique conformément à sa booléenne ;
   calculer (140) une autocorrélation (R) en se basant sur les valeurs binaires ; et
   déterminer (150) une périodicité (P) du signal physiologique en se basant sur l'autocorrélation calculée ;
   dans lequel la détermination d'une périodicité du signal physiologique en se basant sur l'autocorrélation calculée comprend les étapes consistant à :

      trouver un premier pic de l'autocorrélation calculée dans un sens allant d'un décalage le plus court à un décalage le plus long ; et
      définir le premier pic en tant que périodicité du signal physiologique.

2. Procédé selon la revendication 1, dans lequel la pluralité de périodicités cibles est en unités de cycles par minute ou de cycles par seconde.

3. Procédé selon la revendication 1, comprenant en outre les étapes consistant à stocker les valeurs binaires dans un tampon circulaire.

4. Procédé selon la revendication 3, dans lequel chaque élément du tampon circulaire est un bit.

5. Procédé selon la revendication 1, dans lequel l'autocorrélation est calculée en utilisant une fonction NOR exclusive qui produit une valeur de 1 lorsque les deux entrées binaires sont égales.

6. Procédé selon la revendication 1, dans lequel l'autocorrélation est calculée pour les décalages qui correspondent à la pluralité de périodicités cibles.

7. Procédé selon la revendication 1, dans lequel l'autocorrélation est une autocorrélation en temps réel.

8. Procédé selon la revendication 1, comprenant en outre l'étape consistant à calculer l'autocorrélation après une durée prédéterminée.

9. Procédé selon la revendication 1, comprenant en outre les étapes consistant à :

   comparer le premier pic à un seuil ; et
   définir le premier pic en tant que périodicité du signal physiologique uniquement si le premier pic ou une différence entre le premier pic et un élément minimal de l'autocorrélation calculée dépasse le seuil.

10. Procédé selon la revendication 9, comprenant en outre l'étape consistant à transmettre la périodicité déterminée du signal physiologique à un dispositif mobile en utilisant une communication sans fil.

11. Support lisible par machine, comprenant des instructions exécutables par un ou plusieurs processeurs pour mettre en œuvre le procédé destiné à déterminer une périodicité d'un signal physiologique selon l'une quelconque des revendications 1 à 10.

12. Dispositif médical destiné à déterminer une périodicité d'un signal physiologique, comprenant le support lisible par machine selon la revendication 11.

13. Dispositif médical selon la revendication 12, dans lequel le dispositif médical est un dispositif médical implantable actif (AIMD).

<u>100</u>

```
┌─────────────────────────────────────────┐
│   providing a plurality of target        │
│              periodicities               │
└─────────────────────────────────────────┘  110
                    ↓
┌─────────────────────────────────────────┐
│   filtering a physiological signal using │
│   a bandpass filter to generate signal   │
│              samples                     │
└─────────────────────────────────────────┘  120
                    ↓
┌─────────────────────────────────────────┐
│   converting the signal samples into     │
│              binary values               │
└─────────────────────────────────────────┘  130
                    ↓
┌─────────────────────────────────────────┐
│   calculating an autocorrelation based   │
│         on the binary values             │
└─────────────────────────────────────────┘  140
                    ↓
┌─────────────────────────────────────────┐
│   determining a periodicity of the       │
│   physiological signal based on the      │
│       calculated autocorrelation         │
└─────────────────────────────────────────┘  150
```

# FIG. 1

## Raw Signal

## Preprocessed Signal

## Binary Conversion

FIG. 2

EP 4 418 987 B1

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030185449 A1

**Non-patent literature cited in the description**

- **ADÁN TORRALBA AYANCE et al.** *Low-cost micro-controlled based wireless heart rate and oxygen saturation monitor*, ISSN 2474-9044